(19) 
**Europäisches Patentamt**
**European Patent Office**
**Office européen des brevets**

(11) **EP 4 710 840 A1**

(12) **EUROPEAN PATENT APPLICATION**
published in accordance with Art. 153(4) EPC

(43) Date of publication:
**18.03.2026 Bulletin 2026/12**

(21) Application number: **24818792.4**

(22) Date of filing: **07.06.2024**

(51) International Patent Classification (IPC):
**A61B 3/10** (2006.01)     **A61B 3/14** (2006.01)
**A61B 3/00** (2006.01)

(86) International application number:
**PCT/CN2024/098173**

(87) International publication number:
**WO 2024/251266 (12.12.2024 Gazette 2024/50)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **07.06.2023 CN 202310673845**

(71) Applicant: **SVision Imaging, Ltd
Shanghai 200085 (CN)**

(72) Inventors:
• **ZANG, Xuan**
**Shanghai 200085 (CN)**
• **WANG, Jiayin**
**Shanghai 200085 (CN)**
• **CHEN, Jun**
**Shanghai 200085 (CN)**
• **WU, Zhilin**
**Shanghai 200085 (CN)**

(74) Representative: **Vogelbruch, Keang
VOGELBRUCH Patentanwaltsgesellschaft mbH
Paul-Gerhardt-Straße 16
40593 Düsseldorf (DE)**

(54) **METHOD AND APPARATUS FOR MEASURING AXIAL LENGTH OF EYE, AND COMPUTER DEVICE**

(57) The present application relates to a method and apparatus for measuring an axial length of an eye, and a computer device. The method comprises: acquiring an optical image on which an OCT optical focus is aligned with an ocular posterior segment of an eye under test, wherein the optical image comprises the cornea and the fovea of the retina of the eye under test; acquiring an anterior segment endpoint and a posterior segment endpoint of the eye under test according to the optical image; and determining the axial length of the eye under test according to the anterior segment endpoint and the posterior segment endpoint. According to the method, the anterior segment endpoint and the posterior segment endpoint of the eye under test are determined, so that the axial length can be determined according to the anterior segment endpoint and the posterior segment endpoint, that is, an optical image comprising a clear retina is acquired, so that the axial length is acquired according to the optical image comprising the clear retina, and the effect of accurately measuring the axial length can still be achieved for serious ophthalmic diseases.

| obtaining an optical image with an OCT optical focus aligning with a posterior segment of an eye to be measured | S201 |
| --- | --- |
| obtaining an anterior segment endpoint and a posterior segment endpoint of the eye to be measured according to the optical image | S202 |
| determining an eye axial length of the eye to be measured according to the anterior segment endpoint and the posterior segment endpoint | S203 |

**FIG. 2**

EP 4 710 840 A1

Description

## CROSS-REFERENCE TO RELATED APPLICATIONS

The present invention claims the priority to the Chinese patent application No. 202310673845.6 filed with the Chinese Patent Office on June 7, 2023, and entitled "METHOD AND APPARATUS FOR MEASURING AXIAL LENGTH OF EYE", the contents of which are incorporated herein by reference in entirety.

## TECHNICAL FIELD

The present invention relates to the technical field of image processing, and particularly to a method and an apparatus for measuring eye axial length, and a computer device.

## BACKGROUND ART

Eye axial length measurement is crucial for the diagnosis or treatment of numerous ophthalmic diseases, such as cataract, refractive error, glaucoma, strabismus, amblyopia, and others.

In related technologies, optical coherence tomography (i.e., OCT) is usually used to measure eye axial length, mainly by arranging the OCT optical focus on the cornea, obtaining optical images including a clear cornea and a local fundus, and then obtaining eye axial length information through the optical images.

However, for relatively severe ophthalmic diseases, the methods in related technologies have the problem of inaccurate eye axial length measurement.

## SUMMARY

Based on this, it is necessary to provide a method and an apparatus for measuring eye axial length, and a computer device for the above technical problem, which are capable of determining the anterior segment endpoint and posterior segment endpoint of an eye to be measured, and further determining the eye axial length of the eye to be measured according to the anterior segment endpoint and the posterior segment endpoint, so that for relatively severe ophthalmic diseases, accurate measurement of the eye axial length can still be achieved.

In a first aspect, an embodiment of the present invention provides a method for measuring eye axial length. The method comprises:

  obtaining an optical image with an OCT optical focus aligning with a posterior segment of an eye to be measured, wherein the optical image comprises a cornea and a fovea of a retina of the eye to be measured;
  obtaining an anterior segment endpoint and a posterior segment endpoint of the eye to be measured according to the optical image; and
determining an eye axial length of the eye to be measured according to the anterior segment endpoint and the posterior segment endpoint.

In one embodiment, the step of obtaining an optical image with an OCT optical focus aligning with a posterior segment of an eye to be measured comprises: obtaining an optical image with an OCT optical focus aligning with a retina of the eye to be measured.

In one embodiment, the step of obtaining an anterior segment endpoint and a posterior segment endpoint of the eye to be measured according to the optical image comprises: inputting the optical image into a pre-trained first neural network model to obtain the anterior segment endpoint and the posterior segment endpoint output by the first neural network model, wherein the first neural network model is trained based on first optical image samples and corresponding labels, and labels of the first optical image samples comprise an anterior segment endpoint mark and a posterior segment endpoint mark.

In one embodiment, the step of obtaining an anterior segment endpoint and a posterior segment endpoint of the eye to be measured according to the optical image comprises:

  obtaining the cornea and a fovea of the retina of the eye to be measured according to the optical image;
  determining the anterior segment endpoint according to the cornea; and
  determining the posterior segment endpoint according to the fovea of the retina.

In one embodiment, the step of obtaining the cornea and a fovea of the retina of the eye to be measured according to the optical image comprises: inputting the optical image into a pre-trained second neural network model to obtain the cornea and the fovea of the retina output by the second neural network model, wherein the second neural network model is trained based on second optical image samples and corresponding labels, and labels of the second optical image samples comprise a cornea mark and a fovea mark of the retina.

In one embodiment, the step of determining the anterior segment endpoint according to the cornea comprises:

  taking a cornea vertex of the cornea as the anterior segment endpoint; or
  taking an intersection point of an OCT scanning center line and an anterior surface of the cornea as the anterior segment endpoint in response to the optical image being obtained by arranging an OCT scanning center to align with the cornea vertex and the fovea of the retina of the eye to be measured.

In one embodiment, the step of determining the posterior segment endpoint according to the fovea of the retina comprises:

> taking the fovea of the retina as the posterior segment endpoint; or
> performing layering on the retina, obtaining a layering result of at least one retinal layer at the fovea of the retina, and determining the posterior segment endpoint according to the layering result.

In one embodiment, the step of determining the posterior segment endpoint according to the layering result comprises:

> selecting a target layer from the layering result and taking a target point in the target layer as the posterior segment endpoint, wherein the target layer includes one of a retinal pigment epithelium-choroid complex layer, a Bowman's layer, and a choroid layer, and the target point is a point on a straight line where the fovea of the retina and the cornea vertex are located; or
> taking an intersection point between the OCT scanning center line and a target layer in the layering result as the posterior segment endpoint in response to the optical image being obtained by arranging an OCT scanning center to align with the cornea vertex and the fovea of the retina of the eye to be measured.

In one embodiment, the step of determining an eye axial length of the eye to be measured according to the anterior segment endpoint and the posterior segment endpoint comprises:

> obtaining an endpoint distance between the anterior segment endpoint and the posterior segment endpoint, and a correction value corresponding to a retinal thickness of the eye to be measured;
> determining an initial eye axial length of the eye to be measured according to the endpoint distance and the correction value corresponding to the retinal thickness; and
> performing refraction correction on the initial eye axial length to obtain the eye axial length of the eye to be measured.

In one embodiment, the correction value corresponding to the retinal thickness is a preset value, a retinal thickness at the fovea of the retina, or a thickness between a retinal anterior surface at the fovea of the retina and the posterior segment endpoint.

In one embodiment, the preset value is between 100 micrometers and 300 micrometers.

In one embodiment, the step of determining an initial eye axial length of the eye to be measured according to the endpoint distance and the correction value corresponding to the retinal thickness comprises:

taking a difference between the endpoint distance and the correction value corresponding to the retinal thickness as the initial eye axial length of the eye to be measured.

In one embodiment, the step of performing refraction correction on the initial eye axial length to obtain the eye axial length of the eye to be measured comprises: performing refraction correction on the initial eye axial length to obtain the eye axial length of the eye to be measured according to a preset refractive index correction coefficient.

In one embodiment, the refractive index correction coefficient includes an average refractive index correction coefficient; and the step of performing refraction correction on the initial eye axial length to obtain the eye axial length of the eye to be measured according to a preset refractive index correction coefficient comprises:

> calculating an eye axial length $L_2$ of the eye to be measured through the following formula:

$$L_2 = k \times L_1,$$

> where k represents the average refractive index correction coefficient and $L_1$ represents the initial eye axial length.

In one embodiment, the average refractive index correction coefficient is between 1.33 and 1.45.

In one embodiment, the step of determining an eye axial length of the eye to be measured according to the anterior segment endpoint and the posterior segment endpoint comprises:

> obtaining multiple structural layers of the eye to be measured between the anterior segment endpoint and the posterior segment endpoint according to the optical image, wherein the multiple structural layers include an anterior surface and a posterior surface of the cornea, an anterior surface of a lens, and a posterior surface of the lens;
> obtaining multiple segment lengths corresponding one-to-one to the cornea, an anterior chamber, the lens, and a vitreous body of the eye to be measured in an eye axial direction according to the multiple structural layers, the anterior segment endpoint, and the posterior segment endpoint; and
> performing refraction correction and summation on the multiple segment lengths to obtain the eye axial length of the eye to be measured.

In one embodiment, the method further includes:

> obtaining an anterior segment mark of the anterior segment endpoint and a posterior segment mark of the posterior segment endpoint according to the optical image; and
displaying an eye axial length according to the anterior segment mark and the posterior segment mark.

In one embodiment, the step of displaying an eye axial length according to the anterior segment mark and the posterior segment mark comprises:

displaying the anterior segment mark and the posterior segment mark;
obtaining position adjustment information after a user adjusts the anterior segment mark and the posterior segment mark; and
determining the eye axial length according to the position adjustment information and displaying the eye axial length.

In a second aspect, the present invention further provides an apparatus for measuring eye axial length. The apparatus comprises:

a first obtaining module configured to obtain an optical image with an OCT optical focus aligning with a posterior segment of an eye to be measured, wherein the optical image comprises a cornea and a fovea of a retina of the eye to be measured;
a second obtaining module configured to obtain an anterior segment endpoint and a posterior segment endpoint of the eye to be measured according to the optical image; and
a length determining module configured to determine an eye axial length of the eye to be measured according to the anterior segment endpoint and the posterior segment endpoint.

In a third aspect, the present invention further provides a computer device. The computer device includes a memory and a processor, wherein the memory stores a computer program, and the processor implements steps in any embodiment in the first aspect when executing the computer program.

The above method and apparatus for measuring eye axial length, and the computer device first obtain an optical image with an OCT optical focus aligning with the posterior segment of an eye to be measured, then obtain an anterior segment endpoint and a posterior segment endpoint of the eye to be measured according to the optical image, and finally determine an eye axial length of the eye to be measured according to the anterior segment endpoint and the posterior segment endpoint. The above method can determine the eye axial length according to the anterior segment endpoint and the posterior segment endpoint, that is, can obtain the eye axial length according to an optical image including a clear retina by determining the anterior segment endpoint and the posterior segment endpoint of the eye to be measured. For relatively severe ophthalmic diseases, accurate measurement of the eye axial length can still be achieved.

## BRIEF DESCRIPTION OF DRAWINGS

FIG. 1 is an application environment diagram of a method for measuring eye axial length in one embodiment;
FIG. 2 is a flow schematic diagram of a method for measuring eye axial length in one embodiment;
FIG. 3 is a schematic diagram of an optical image with an optical focus aligned with a cornea of an eye to be measured in one embodiment;
FIG. 4 is a schematic diagram of an optical image with an optical focus aligned with a retina of an eye to be measured in one embodiment;
FIG. 5 is a schematic diagram of a corneal reflection spot and a cornea vertex in one embodiment;
FIG. 6 is a schematic diagram of a user interaction interface in one embodiment;
FIG. 7 is a flow schematic diagram of determining an anterior segment endpoint and a posterior segment endpoint in one embodiment;
FIG. 8 is a flow schematic diagram of determining an eye axial length in one embodiment;
FIG. 9 is a schematic diagram of an endpoint distance between an anterior segment endpoint and a posterior segment endpoint in one embodiment;
FIG. 10 is a schematic diagram of various structural surfaces between an anterior segment endpoint and a posterior segment endpoint in one embodiment;
FIG. 11 is a flow schematic diagram of a method for measuring eye axial length in another embodiment;
FIG. 12 is a structural schematic diagram of an apparatus for measuring eye axial length in one embodiment;
FIG. 13 is a structural schematic diagram of an apparatus for measuring eye axial length in another embodiment;
FIG. 14 is a structural schematic diagram of an apparatus for measuring eye axial length in another embodiment; and
FIG. 15 is an internal structural diagram of a computer device in one embodiment.

## DETAILED DESCRIPTION OF EMBODIMENTS

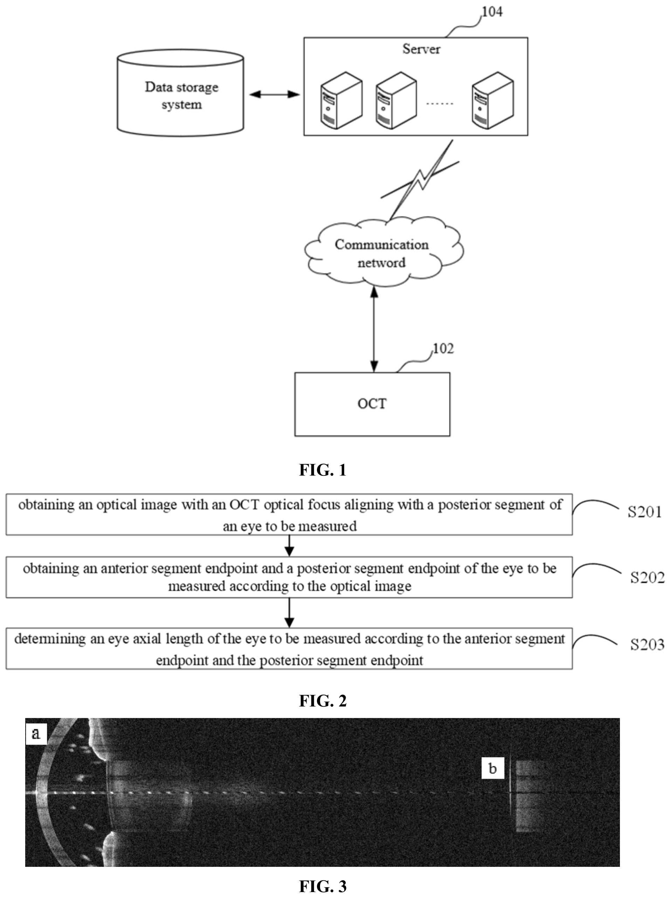

In order to make the objective, technical solutions, and advantages of the present invention clearer and more understandable, the present invention is described in further detail hereinafter in conjunction with the accompanying drawings and embodiments. It should be understood that the specific embodiments described herein are for the sole purpose of explaining the present invention and are not intended to limit the present invention.
Embodiments of the present invention provide a method for measuring eye axial length, which can be applied to an application environment shown in FIG. 1. In the application environment, the OCT 102 communicates with the server 104 through a network. A data storage system can store data that needs to be processed by the server 104. Optionally, the OCT 102 is configured to acquire an optical image of an eye to be measured, and can be a time-domain OCT, a frequency-domain OCT, or a swept-source OCT. The data storage system can be integrated in the OCT 102, or can be placed on the server 104, or on a cloud, or on another network server. The server 104 can be implemented by using an independent server or a server cluster composed of multiple servers.

In one embodiment, as shown in FIG. 2, the method for measuring eye axial length is provided. An example that the method applies to the server 104 in FIG. 1 is taken, and the method includes following steps.

S201: obtaining an optical image with an OCT optical focus aligning with a posterior segment of an eye to be measured.

Optionally, the OCT can be controlled to perform scanning on the eye to be measured, so as to obtain an optical image of the eye to be measured. Further, the OCT sends the obtained optical image to the server, and the server can acquire the optical image of the eye to be measured.

It needs to be explained that, in related art, the OCT optical focus is placed on a cornea, an optical image including a clear cornea and a partial fundus is acquired, and axial length information is acquired through the optical image. As shown in FIG. 3, an optical image scanned with the OCT optical focus aligning with the cornea of the eye to be measured can show a relatively clear cornea a and a partial retina b. For relatively severe ophthalmic diseases, if still using the OCT to arrange the optical focus to align with the cornea, a retina in the optical image obtained in this manner will be unclear, leading to an inability to accurately measure eye axial length.

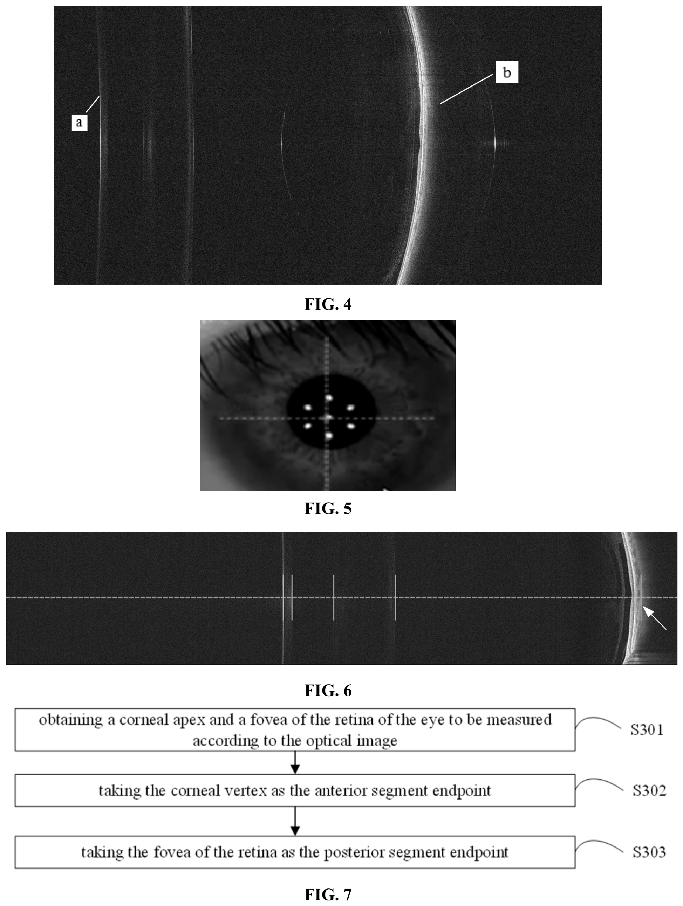

Based on this, in this solution, when acquiring the optical image of the eye to be measured, by aligning the OCT optical focus with the posterior segment, an optical image including a clear retina can be acquired, as shown in FIG. 4, and then, an eye axial length of the eye to be measured can be determined according to the optical image including the clear retina. For acquiring the optical image including the clear retina, the OCT can scan a human eye, and a reference arm can be adjusted in real time based on an obtained preview image, so that a retina resolution in the image meets the requirement, thereby obtaining the above optical image. Specifically, an artificial determination can be made on whether the retina resolution in the image meets the requirement; or the retina resolution in the image can be acquired, and an automatic determination can be performed by determining whether the retina resolution reaches a preset resolution threshold. In addition, when the retina resolution in the image reaches the resolution threshold, a reminder indicating that the retina resolution meets the requirement can be sent, such as a voice prompt and/or a text prompt. The resolution threshold can be set according to actual needs, which is not limited here. When the retina image resolution meets the requirement, it can be considered that the OCT optical focus aligns with the posterior segment of the eye to be measured.

The above posterior segment can include a vitreous body and a posterior structure of the vitreous body along the eye axial direction, such as a retina. In one possible implementation, the step of obtaining an optical image with an OCT optical focus aligning with a posterior segment of an eye to be measured comprises: obtaining an optical image with an OCT optical focus aligning with a retina of the eye to be measured.

Exemplarily, when acquiring the optical image of the eye to be measured, an OCT scanning center is arranged to align with a cornea vertex of the eye to be measured, and simultaneously, the OCT optical focus is arranged to align with the retina of the eye to be measured; and then, scanning is performed on the eye to be measured. After the OCT scanning center aligns with the cornea vertex of the eye to be measured, an initial imaging exists in a display screen, and whether the initial imaging includes a clear retina and a fovea of the retina is observed. If a deviation is found, that is, the initial imaging does not include a clear retina and/or a fovea of the retina, then the OCT scanning center is further adjusted to align with the cornea vertex and the fovea of the retina of the eye to be measured based on the initial imaging; if no deviation exists, an acquisition button is touched to acquire a current image, so as to obtain a target optical image, i.e., the optical image in which the OCT scanning center aligns with the cornea vertex of the eye to be measured. The optical image at least includes a cornea, a retina, and a fovea of the retina of the eye to be measured.

Optionally, a fixation light can be used for adjusting the OCT scanning center to align with the fovea of the retina. The fixation light is turned on, and through visible light emitted by the fixation light, a fixation direction of a patient is adjusted, so as to adjust a position of the fovea of the retina, thereby enabling the OCT scanning center to align with the retinal fovea. A structure light in the OCT is projected to a cornea in the initial imaging, and a corneal reflection spot formed by corneal reflection is acquired. A cornea vertex of the eye to be measured is determined according to a position of the corneal reflection spot. Further, the OCT scanning center is arranged to align with the cornea vertex of the eye to be measured. For example, as shown in FIG. 5, a center point of the corneal reflection spot is the cornea vertex, and then the OCT scanning center is arranged to align with the cornea vertex of the eye to be measured. The OCT device can be adjusted to align the corneal reflection spot with a center of a pupil camera, and an operation distance is adjusted, so that the corneal reflection spot is imaged most clearly. At this time, the cornea vertex can be determined on the image.

It needs to be explained that the OCT scanning center does not need to align with a cornea vertex of the eye to be measured, as long as the optical image can completely show a cornea, a clear retina, and a fovea of the retina of the eye to be measured, that is, the optical image can allow a certain degree of offset between the OCT scanning center and the cornea vertex.

S202: obtaining an anterior segment endpoint and a posterior segment endpoint of the eye to be measured according to the optical image.

In embodiments of the present invention, the anterior segment endpoint represents an endpoint at an anterior segment of the eye to be measured, which can be determined according to a cornea, or can be determined according to an OCT scanning center line and the cornea. The posterior segment endpoint represents an endpoint at a posterior segment of the eye to be measured, which can be determined according to a fovea of the retina, or can be determined according to a scanning center line and a retina. When the OCT scanning center aligns with a cornea vertex and a fovea of the retina of the eye to be measured, in the acquired optical image, the cornea vertex and the fovea of the retina are located in a middle portion of the image, and the OCT scanning center line is a center line of the optical image.

In a possible implementation, a cornea and a fovea of the retina of the eye to be measured are obtained, where the cornea vertex is used as the anterior segment endpoint, and the fovea of the retina is used as the posterior segment endpoint.

In another possible implementation, a cornea and a fovea of the retina of the eye to be measured is obtained, where the cornea vertex is used as the anterior segment endpoint; layering is performed on the retina, so as to acquire at least one retinal layer; and a point on one retinal layer of the acquired retinal layers is used as the posterior segment endpoint of the eye to be measured, wherein the point is located on a straight line passing through the fovea of the retina and the cornea vertex. Each retinal layer refers to a common edge between adjacent tissue structures. The straight line passing through the fovea of the retina and the cornea vertex can be considered a straight line in the eye axial direction.

In yet another possible implementation, a cornea and a retina of the eye to be measured are obtained, where an intersection point between the OCT scanning center line and the cornea of the eye to be measured is used as the anterior segment endpoint of the eye to be measured, and an intersection point between the OCT scanning center line and the retina of the eye to be measured is used as the posterior segment endpoint of the eye to be measured. For example, the method can include obtaining a cornea and a fovea of the retina of the eye to be measured; using an intersection point between the OCT scanning center line and an anterior surface of the cornea of the eye to be measured as the anterior segment endpoint of the eye to be measured;

performing layering on the retina to acquire at least one retinal layer, and using an intersection point between the OCT scanning center line and one retinal layer of the acquired retinal layers at the fovea of the retina of the eye to be measured as the posterior segment endpoint of the eye to be measured.

In yet another possible implementation, an optical image of the eye to be measured is input into a pre-trained first neural network model, so that the model outputs the anterior segment endpoint and the posterior segment endpoint of the eye to be measured. The first neural network model is trained based on first optical image samples and corresponding labels, and the labels of the first optical image samples include an anterior segment endpoint mark and a posterior segment endpoint mark.

S203: determining an eye axial length of the eye to be measured according to the anterior segment endpoint and the posterior segment endpoint.

In the embodiment of the present invention, the eye axial length represents a length of an eyeball of the eye to be measured from front to back.

Optionally, a determination logic, which is predefined and used to determine an eye axial length, can be used to determine the eye axial length of the eye to be measured according to the anterior segment endpoint and the posterior segment endpoint.

In a possible implementation, an endpoint distance between the anterior segment endpoint and the posterior segment endpoint can be measured, and the endpoint distance between the anterior segment endpoint and the posterior segment endpoint is used as the eye axial length of the eye to be measured.

After determining the eye axial length, the eye axial length needs to be further displayed. Different measurement methods of the eye axial length correspond to different display manners of the eye axial length. A display manner of the eye axial length in the present invention can include the following steps: acquiring an optical image in which the OCT scanning center aligns with a pupil center of the eye to be measured; obtaining an anterior segment endpoint and a posterior segment endpoint of the eye to be measured according to the optical image; determining an eye axial length of the eye to be measured according to the anterior segment endpoint and the posterior segment endpoint; obtaining an anterior segment mark of the anterior segment endpoint and a posterior segment mark of the posterior segment endpoint according to the optical image; and displaying an eye axial length according to the anterior segment mark and the posterior segment mark.

Optionally, the eye axial length can be displayed directly according to the positions of the current anterior segment mark and current posterior segment mark. Since there can be multiple positions for the posterior segment endpoint, further, according to different positions of the posterior segment endpoint, multiple eye axial lengths can exist. Based on this, an optional manner for displaying different eye axial lengths is provided, including: displaying the anterior segment mark and the posterior segment mark; obtaining position adjustment information after a user adjusts the anterior segment mark and the posterior segment mark; and determining the eye axial length according to the position adjustment information and displaying the eye axial length.

Referring to a schematic diagram of a user interaction interface shown in FIG. 6, an arrow indicates a scleral segmentation line. When a user adjusts the posterior segment mark, the posterior segment mark cannot move right beyond the scleral segmentation line. The scleral segmentation line can prompt a user for an adjustment range of the posterior segment mark, preventing the user from excessively moving the posterior segment mark.

It needs to be explained that, by adjusting the anterior segment mark and the posterior segment mark by the user, different eye axial lengths can be displayed, satisfying different needs of the user regarding displaying the eye axial lengths.

In the method for measuring eye axial length provided in embodiments of the present invention, an optical image with an OCT optical focus aligning with the posterior segment of an eye to be measured is first obtained, then an anterior segment endpoint and a posterior segment endpoint of the eye to be measured is obtained according to the optical image, and finally, an eye axial length of the eye to be measured is determined according to the anterior segment endpoint and the posterior segment endpoint. The above method can determine the eye axial length according to the anterior segment endpoint and the posterior segment endpoint, that is, can obtain the eye axial length according to an optical image including a clear retina by determining the anterior segment endpoint and the posterior segment endpoint of the eye to be measured. For relatively severe ophthalmic diseases, accurate measurement of the eye axial length can still be achieved.

The eye axial length represents a length of the eyeball from front to back. Measuring the eye axial length requires determining an anterior endpoint and a posterior endpoint of the eyeball, that is, an anterior segment endpoint and a posterior segment endpoint in the present invention. In order to accurately measure the eye axial length, it is necessary to precisely determine the anterior segment endpoint and the posterior segment endpoint. Based on this, in one embodiment, an optional manner for determining the anterior segment endpoint and the posterior segment endpoint of the eye to be measured is provided. As shown in FIG. 7, it can include the following steps:

S301, obtaining a cornea vertex and a fovea of the retina of the eye to be measured according to the optical image;
S302, taking the cornea vertex as the anterior segment endpoint; and
S303, taking the fovea of the retina as the posterior segment endpoint.

In the embodiment of the present invention, the fovea of the retina represents the position of a fovea centralis of the retina.

In a possible implementation, a cornea vertex and a fovea of the retina of the eye to be measured can be obtained by a pre-configured neural network model, where the cornea vertex is used as the anterior segment endpoint, and the fovea of the retina is used as the posterior segment endpoint. Specifically, the optical image can be input into a pre-trained second neural network model to obtain the cornea and the fovea of the retina output by the second neural network model, wherein the cornea includes the cornea vertex or the anterior surface of the cornea. The second neural network model is trained based on the second optical image samples and corresponding labels, and labels of the second optical image samples include a cornea mark and a fovea mark of the retina. It needs to be noted that the second optical image samples can be different from the first optical image samples; or the second optical image samples can be the same as the first optical image samples but using different labels.

In addition, since relevant technologies do not provide very clear definitions for the anterior segment endpoint and the posterior segment endpoint, different technicians can have differences in determining the anterior segment endpoint and the posterior segment endpoint. Based on this, in present invention, a manner for determining the anterior segment endpoint and the posterior segment endpoint also can be: determining the OCT scanning center line as a center line of the optical image, and taking an intersection point of an OCT scanning center line and an anterior surface of the cornea as the anterior segment endpoint in response to the optical image being obtained by arranging an OCT scanning center to align with the cornea vertex and the fovea of the retina of the eye to be measured; and performing layering on the retina, obtaining a layering result of at least one retinal layer at the fovea of the retina, and determining the posterior segment endpoint according to the layering result.

Optionally, the cornea includes an anterior surface of the cornea and a posterior surface of the cornea. The cornea can be subjected to layering processing to obtain the anterior surface of the cornea; and further, an intersection point between the OCT scanning center line and the anterior surface of the cornea is used as the anterior segment endpoint.

Optionally, the retina can be subjected to layering in the following manner to obtain a layering result of at least one retinal layer at the fovea of the retina: inputting the optical image into a pre-trained third neural network model to obtain the layering result output by the third neural network model, wherein the third neural network model is trained based on third optical image samples and corresponding labels, and labels of the third optical image samples include a retinal layer mark at the fovea of the retina. It needs to be noted that the third optical image samples can be different from the first optical image samples and the second optical image samples; or the third optical image samples can be the same as the first optical image samples and/or the second optical image samples, but using different labels.

Further, in a possible implementation, the step of determining the posterior segment endpoint according to the layering result can include: selecting a target layer from the layering result and taking a target point in the target layer as the posterior segment endpoint, wherein the target layer includes one of a retinal pigment epithelium-choroid complex layer, a Bowman's layer, and a choroid layer, and the target point is a point on a straight line where the fovea of the retina and the cornea vertex are located.

In another possible implementation, the step of determining the posterior segment endpoint according to the layering result can include: taking an intersection point between the OCT scanning center line and one target layer in the layering result as the posterior segment endpoint in response to the optical image being obtained by arranging an OCT scanning center to align with the cornea vertex and the fovea of the retina of the eye to be measured. For example, the layering is performed on the retina to obtain at least one retinal layer at the fovea of the retina among a retinal pigment epithelium-choroid complex layer, a Bowman's layer, and a choroid layer; and an intersection point between the OCT scanning center line and one of the retinal layers is used as the posterior segment endpoint.

Exemplarily, the optical image can be input into a pre-trained third neural network model, and the model performs layering on the retina in the optical image to obtain retinal layers at the fovea of the retina, including the retinal pigment epithelium-choroid complex layer, the Bowman's layer, and the choroid layer; then, an intersection point between the OCT scanning center line and any one of the above retinal layers is used as the posterior segment endpoint. It needs to be explained that there are different methods for measuring the eye axial length, such as ultrasonic measurement and optical biometry measurement. Different measurement methods of the eye axial length correspond to different eye axial lengths. For example, the eye axial length measured by ultrasonic measurement is a length from a corneal surface layer to the inner boundary of the retina, and the eye axial length measured by optical biometry measurement is a length from a tear film surface layer to the retinal pigment epithelium layer. That is, different measurement manners correspond to different manners for determining the anterior segment endpoint and the posterior segment endpoint. In the present invention, by using OCT to measure the eye axial length, the anterior segment endpoint can be the cornea vertex, and the posterior segment endpoint can be the fovea of the retina; alternatively, the anterior segment endpoint can be an intersection point between the anterior surface of the cornea and the OCT scanning center line. The posterior segment endpoint can be an intersection point between the OCT scanning center line and any one of the layers at the fovea of the retina. For example, the posterior segment endpoint can be an intersection point between the OCT scanning center line and the choroid layer at the fovea of the retina.

In the embodiment of the present invention, an optional manner is provided for quickly determining the anterior segment endpoint and the posterior segment endpoint of the eye to be measured, which includes obtaining the cornea vertex and the fovea of the retina of the eye to be measured, taking the cornea vertex as the anterior segment endpoint, and taking the fovea of the retina as the posterior segment endpoint. This provides data support for subsequently determining the eye axial length of the eye to be measured.

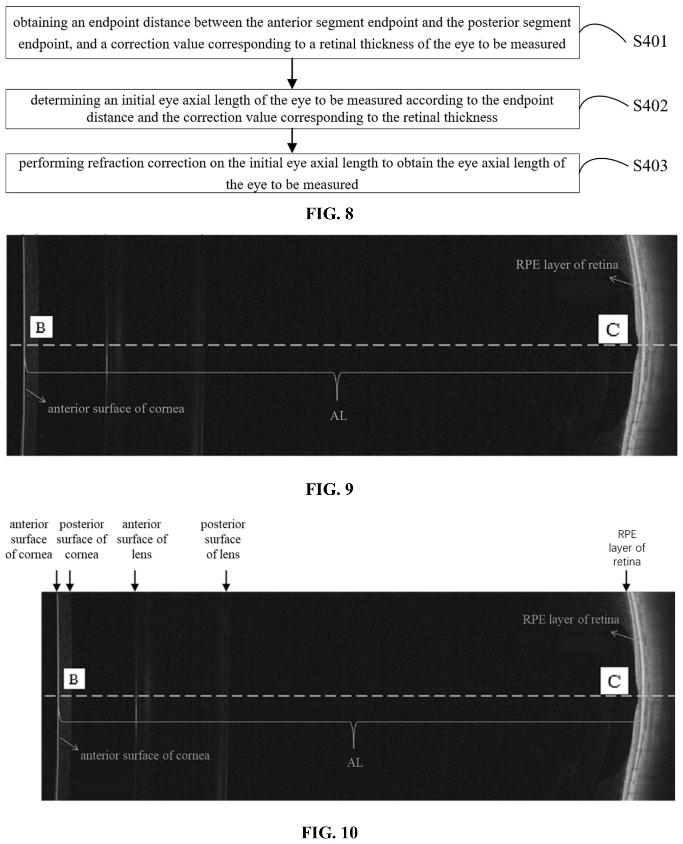

In one embodiment, based on the above embodiments, an optional manner for determining the eye axial length is provided. As shown in FIG. 8, it can include the following steps.

S401: obtaining an endpoint distance between the anterior segment endpoint and the posterior segment endpoint, and a correction value corresponding to a retinal thickness of the eye to be measured.

Optionally, the correction value corresponding to the retinal thickness can be a preset value, can be a retinal thickness at the fovea of the retina, or can be a thickness between a retinal anterior surface at the fovea of the retina and the posterior segment endpoint. The preset value can be set based on statistical results of the retinal thickness at the fovea of the retina. In embodiments of the present invention, the retinal thickness of the eye to be measured can be 200 $\mu$m, and the preset value can be between 100 $\mu$m and 300 $\mu$m, for example, the preset value is 200 $\mu$m. It needs to be noted that the retinal thickness of different eyes to be measured can be different.

Optionally, after determining the anterior segment endpoint and the posterior segment endpoint, the distance between the anterior segment endpoint and the posterior segment endpoint can be directly measured. As shown in FIG. 9, point B represents the anterior segment endpoint, point C represents the posterior segment endpoint, and AL represents the endpoint distance between the anterior segment endpoint and the posterior segment endpoint.

S402: determining an initial eye axial length of the eye to be measured according to the endpoint distance and the correction value corresponding to the retinal thickness.

In a possible implementation, a difference between the endpoint distance and the correction value corresponding to the retinal thickness can be used as the initial eye axial length of the eye to be measured, that is, initial eye axial length = endpoint distance - correction value.

Optionally, since the retina of the eye to be measured has a certain thickness, which may affect the accuracy of the measurement of the eye axial length, it is necessary to process the retinal thickness, that is, subtract the corresponding correction value for the retinal thickness, to ensure the accuracy of the measurement of the eye axial length.

S403: performing refraction correction on the initial eye axial length to obtain the eye axial length of the eye to be measured.

Optionally, since the eye contains a large amount of fluid, light is refracted when passing through the eye, which has an impact on the measurement of the eye axial length; and therefore, refractive correction needs to be performed to obtain an accurate and true eye axial length.

In a possible implementation, the refraction correction can be performed on the initial eye axial length to obtain the eye axial length of the eye to be measured according to a preset refractive index correction coefficient.

As shown in FIG. 10, the eye includes structures such as a cornea, a lens, and a retina. In the eye, the refractive indices of different structures are slightly different. A refractive index at the cornea (that is, between an anterior surface of the cornea and a posterior surface of the cornea), a refractive index at the anterior chamber (that is, between the posterior surface of the cornea and an anterior surface of the lens), a refractive index at the lens (that is, between the anterior surface of the lens and a posterior surface of the lens), and a refractive index at the vitreous body (that is, between the posterior surface of the lens and an anterior surface of the retinal RPE layer) are all slightly different. To accurately calculate the eye axial length, the step of performing refractive correction on the initial eye axial length according to refractive index correction coefficients can include: correcting a length between the anterior surface of the cornea and the posterior surface of the cornea using a first refractive index correction coefficient; correcting a length between the posterior surface of the cornea and the anterior surface of the lens using a second refractive index correction coefficient; correcting a length between the anterior surface of the lens and the posterior surface of the lens using a third refractive index correction coefficient; correcting a length between the posterior surface of the lens and the anterior surface of the retinal RPE layer using a fourth refractive index correction coefficient; and summing the corrected lengths to obtain the eye axial length of the eye to be measured. The first refractive index correction coefficient, the second refractive index correction coefficient, the third refractive index correction coefficient, and the fourth refractive index correction coefficient are empirical values and are all stored in the system in advance.

Optionally, considering that proportions of lengths of various eye structures are usually similar among different persons, proportion values for various structures of the eye can be preset to determine segment lengths corresponding to different structures, and segment correction can then be performed for the segment lengths. Based on this, in a possible implementation, the refractive index correction coefficient includes a first refractive index correction coefficient corresponding to the cornea, a second refractive index correction coefficient corresponding to the anterior chamber, a third refractive index correction coefficient corresponding to the lens, and a fourth refractive index correction coefficient corresponding to the vitreous body. The eye axial length of the eye to be measured can be obtained in the following manner:

first, determining multiple segment lengths corresponding one-to-one to the refractive index correction coefficients according to the initial eye axial length and preset proportion data, wherein the proportion data include a first proportion value corresponding to the cornea, a second proportion value corresponding to the anterior chamber, a third proportion value corresponding to the lens, and a fourth proportion value corresponding to the vitreous body, and a sum of the first proportion value, the second proportion value, the third proportion value, and the fourth proportion value is 1;
then, calculating an eye axial length $L_2$ of the eye to be measured through the following formula:

$$L_2 = k_1 \times L_{11} + k_2 \times L_{12} + k_3 \times L_{13} + k_4 \times L_{14},$$

where $k_1$ represents the first refractive index correction coefficient corresponding to segment length $L_{11}$, $k_2$ represents the second refractive index correction coefficient corresponding to segment length $L_{12}$, $k_3$ represents the third refractive index correction coefficient corresponding to segment length $L_{13}$, and $k_4$ represents the fourth refractive index correction coefficient corresponding to segment length $L_{14}$.

To speed up the calculation, an average refractive index correction coefficient can be calculated for the refractive index correction coefficients corresponding to structures of the eye, and based on the average refractive index correction coefficient, the eye axial length of the eye to be measured can be quickly calculated. Based on this, in another possible implementation, the refractive index correction coefficient includes an average refractive index correction coefficient. The eye axial length of the eye to be measured can be obtained in the following manner:

calculating an eye axial length $L_2$ of the eye to be measured through the following formula:

$$L_2 = k \times L_1,$$

where k represents the average refractive index correction coefficient and $L_1$ represents the initial eye axial length.

Optionally, the average refractive index correction coefficient can be between 1.33 and 1.45, for example, the average refractive index correction coefficient is 1.41.

In the embodiment of the present invention, an optional manner for determining the eye axial length is provided. The initial eye axial length is determined through an endpoint distance between an anterior segment endpoint and a posterior segment endpoint, and a retinal thickness of the eye to be measured, and the initial eye axial length is further subjected to refractive correction to ensure authenticity and accuracy of the eye axial length.

In another embodiment, based on the above embodiments, another optional manner for determining the eye axial length is provided, which includes the following steps.

Step a1: obtaining multiple structural layers of the eye to be measured between the anterior segment endpoint and the posterior segment endpoint according to the optical image, wherein the multiple structural layers include an anterior surface and a posterior surface of the cornea, an anterior surface of a lens, and a posterior surface of the lens.

A structure layer recognition can be performed on the optical image based on a pre-trained neural network model to obtain multiple structure layers located between the anterior segment endpoint and the posterior segment endpoint. In practical implementation, the optical image can be input into a pre-trained fourth neural network model to obtain multiple structure layers output by the fourth neural network model, wherein the fourth neural network model is trained based on fourth optical image samples and corresponding labels, and labels of the fourth optical image samples include multiple structural layer marks. It should be noted that the fourth optical image samples can be different from the above three types of optical image samples, or can be the same as one or more of the first optical image samples, the second optical image samples, and the third optical image samples, but using different labels.

Step a2: obtaining multiple segment lengths corresponding one-to-one to the cornea, an anterior chamber, the lens, and a vitreous body of the eye to be measured in an eye axial direction according to the multiple structural layers, the anterior segment endpoint, and the posterior segment endpoint.

Optionally, the multiple segment lengths can be obtained in the following manner: first determining an intersection point of each structure layer with a target connection line, wherein the target connection line is a connection line between the anterior segment endpoint

and the posterior segment endpoint; and then obtaining distances between two adjacent points among the anterior segment endpoint, the posterior segment endpoint and each intersection point to obtain the multiple segment lengths.

Step a3: performing refraction correction and summation on the multiple segment lengths to obtain the eye axial length of the eye to be measured.

Optionally, when calculating the eye axial length of the eye to be measured, each segment length can be subjected to refractive correction first, and then the corrected length values are summed to obtain the eye axial length. For each segment length, a corrected length value can be calculated using the following formula:

$$L'_{1i} = k_i \times L_{1i},$$

where $L'_{1i}$ represents the corrected length value corresponding to the i-th segment length $L_{1i}$, and $k_i$ represents a refractive index correction coefficient corresponding to the i-th segment length $L_{1i}$.

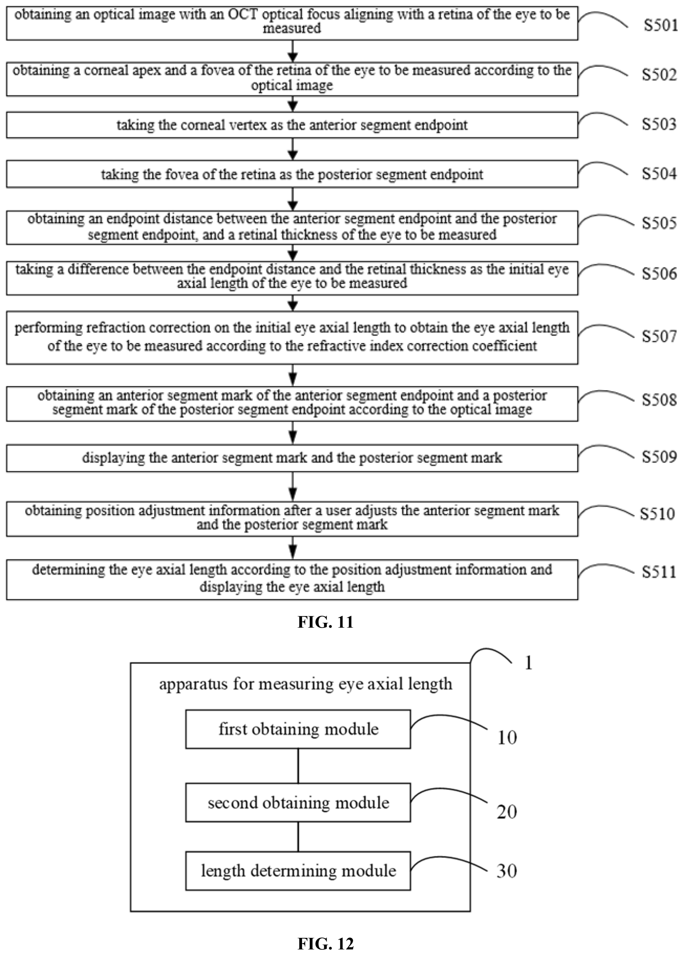

In addition, in one embodiment, an optional example of the method for measuring eye axial length is further provided in the embodiment of the present invention. As shown in FIG. 11, the method includes the following steps.

S501: obtaining an optical image with an OCT optical focus aligning with a retina of the eye to be measured,

wherein the optical image comprises a cornea and a fovea of a retina of the eye to be measured.

S502: obtaining a cornea vertex and a fovea of the retina of the eye to be measured according to the optical image.

S503: taking the cornea vertex as the anterior segment endpoint.

Optionally, the intersection point between the OCT scanning center line and the anterior surface of the cornea can also be used as the anterior segment endpoint.

S504: taking the fovea of the retina as the posterior segment endpoint.

Optionally, the step of determining the posterior segment endpoint according to the OCT scanning center line and the fovea of the retina can comprise: performing the layering on the retina to obtain at least one retinal layer at the fovea of the retina among a retinal pigment epithelium-choroid complex layer, a Bowman's layer, and a choroid layer; using an intersection point between the OCT scanning center line and one of the retinal layers as the posterior segment endpoint.

S505: obtaining an endpoint distance between the anterior segment endpoint and the posterior segment endpoint, and a retinal thickness of the eye to be measured.

S506: taking a difference between the endpoint distance and the retinal thickness as the initial eye axial length of the eye to be measured.

S507: performing refraction correction on the initial eye axial length to obtain the eye axial length of the eye to be measured according to the refractive index correction coefficient.

S508: obtaining an anterior segment mark of the anterior segment endpoint and a posterior segment mark of the posterior segment endpoint according to the optical image.

S509: displaying the anterior segment mark and the posterior segment mark.

S510: obtaining position adjustment information after a user adjusts the anterior segment mark and the posterior segment mark.

S511: determining the eye axial length according to the position adjustment information and displaying the eye axial length.

The process of S501-S511 above can refer to the description of the above method embodiments, and its implementation principle and technical effect are similar and will not be repeated here.

It should be understood that although steps in the flowcharts involved in the above embodiments are displayed sequentially in accordance with arrows, these steps are not necessarily performed sequentially in accordance with the arrows. Unless explicitly stated herein, execution of these steps is not strictly limited in order, and these steps can be executed in other orders. In addition, at least a part of the steps in the flowcharts involved in the above embodiments can include multiple steps or multiple stages, and these steps or stages are not necessarily completed at the same moment but can be executed at different moments. The execution order of these steps or stages is not necessarily sequential. Instead, they may be carried out alternately or in rotation with other steps, or with at least a portion of the steps or phases within other steps.

Based on the same inventive concept, the embodiments of the present invention further provide an apparatus for measuring eye axial length for implementing the above method for measuring eye axial length. A solution for solving problems provided by the apparatus is similar to the solution recorded in the above method, so specific limitations in one or more embodiments of the apparatus for measuring eye axial length provided below can refer to limitations of the method for measuring eye axial length described above and will not be repeated here.

In one embodiment, as shown in FIG. 12, the present invention provides an apparatus for measuring eye axial length 1, including: a first obtaining module 10, a second obtaining module 20, and a length determining module 30.

The first obtaining module 10 is configured to obtain an optical image with an OCT optical focus aligning with a posterior segment of an eye to be measured, wherein the optical image comprises a cornea and a fovea of a retina of the eye to be measured.

The second obtaining module 20 is configured to obtain an anterior segment endpoint and a posterior segment endpoint of the eye to be measured according to the optical image.

The length determining module 30 is configured to determine an eye axial length of the eye to be measured according to the anterior segment endpoint and the posterior segment endpoint.

In one embodiment, the first obtaining module 10 above can further be configured to: obtain an optical image with an OCT optical focus aligning with a retina of the eye to be measured.

In one embodiment, the second obtaining module 20 above can further be configured to: input the optical image into a pre-trained first neural network model to obtain the anterior segment endpoint and the posterior segment endpoint output by the first neural network model, wherein the first neural network model is trained based on first optical image samples and corresponding labels, and labels of the first optical image samples include an anterior segment endpoint mark and a posterior segment endpoint mark.

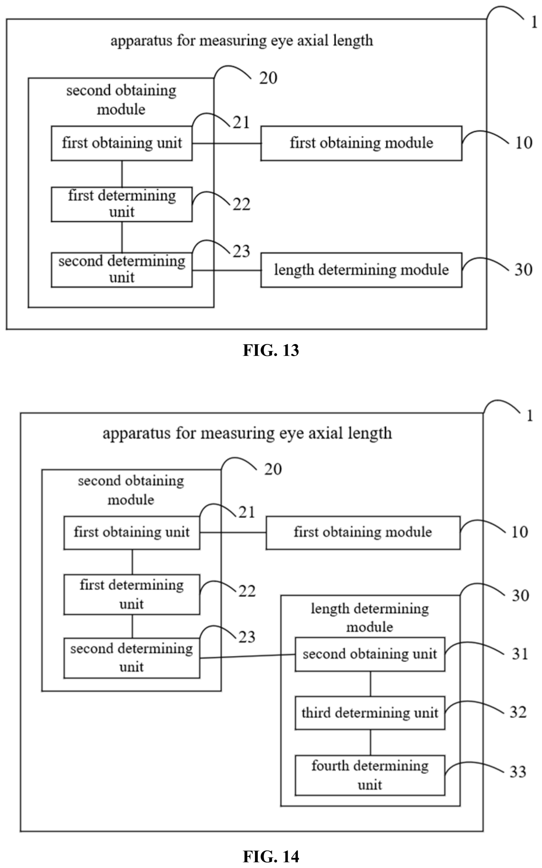

In one embodiment, as shown in FIG. 13, the second obtaining module 20 above includes:

a first obtaining unit 21, configured to obtain the cornea and a fovea of the retina of the eye to be measured according to the optical image;
a first determining unit 22, configured to determine the anterior segment endpoint according to the cornea; and
a second determining unit 23, configured to determine the posterior segment endpoint according to the fovea of the retina.

In one embodiment, the first obtaining unit 21 above can further be configured to: input the optical image into a pre-trained second neural network model to obtain the cornea and the fovea of the retina output by the second neural network model, wherein the second neural network model is trained based on second optical image samples and corresponding labels, and labels of the second optical image samples include a cornea mark and a fovea mark of the retina.

In one embodiment, the first determining unit 22 above can further be configured to:

take a cornea vertex of the cornea as the anterior segment endpoint; or
take an intersection point of an OCT scanning center line and an anterior surface of the cornea as the anterior segment endpoint in response to the optical image being obtained by arranging an OCT scanning center to align with the cornea vertex and the fovea of the retina of the eye to be measured.

The second determining unit 23 above can further be configured to:

take the fovea of the retina as the posterior segment endpoint; or

perform layering on the retina, obtaining a layering result of at least one retinal layer at the fovea of the retina, and determining the posterior segment endpoint according to the layering result.

In one embodiment, the second determining unit 23, when executing the step of determining the posterior segment endpoint according to the layering result, can further be configured to:

select a target layer from the layering result and take a target point in the target layer as the posterior segment endpoint, wherein the target layer includes one of a retinal pigment epithelium-choroid complex layer, a Bowman's layer, and a choroid layer, and the target point is a point on a straight line where the fovea of the retina and the cornea vertex are located; or

take an intersection point between the OCT scanning center line and a target layer in the layering result as the posterior segment endpoint in response to the optical image being obtained by arranging an OCT scanning center to align with the cornea vertex and the fovea of the retina of the eye to be measured.

In one embodiment, as shown in FIG. 14, the length determining module 30 above includes:

a second obtaining unit 31, configured to obtain an endpoint distance between the anterior segment endpoint and the posterior segment endpoint, and a correction value corresponding to a retinal thickness of the eye to be measured;

a third determining unit 32, configured to determine an initial eye axial length of the eye to be measured according to the endpoint distance and the correction value corresponding to the retinal thickness; and

a fourth determining unit 33, configured to perform refraction correction on the initial eye axial length to obtain the eye axial length of the eye to be measured.

In one embodiment, the correction value corresponding to the retinal thickness is a preset value, a retinal thickness at the fovea of the retina, or a thickness between a retinal anterior surface at the fovea of the retina and the posterior segment endpoint.

In one embodiment, the preset value is between 100 micrometers and 300 micrometers.

In one embodiment, the third determining unit 32 above can be configured to:

take a difference between the endpoint distance and the correction value corresponding to the retinal thickness as the initial eye axial length of the eye to be measured.

In one embodiment, the fourth determining unit 33 above can be configured to:

perform refraction correction on the initial eye axial length to obtain the eye axial length of the eye to be measured according to a preset refractive index correction coefficient.

In one embodiment, the refractive index correction coefficient includes an average refractive index correction coefficient; and the fourth determining unit 33 above can further be configured to:

calculate an eye axial length $L_2$ of the eye to be measured through the following formula:

$$L_2 = k \times L_1,$$

where k represents the average refractive index correction coefficient and $L_1$ represents the initial eye axial length.

In one embodiment, the average refractive index correction coefficient is between 1.33 and 1.45.

In one embodiment, the length determining module 30 above can include:

a third obtaining unit, configured to obtain multiple structural layers of the eye to be measured between the anterior segment endpoint and the posterior segment endpoint according to the optical image, wherein the multiple structural layers include an anterior surface and a posterior surface of the cornea, an anterior surface of a lens, and a posterior surface of the lens;

a fourth obtaining unit, configured to obtain multiple segment lengths corresponding one-to-one to the cornea, an anterior chamber, the lens, and a vitreous body of the eye to be measured in an eye axial direction according to the multiple structural layers, the anterior segment endpoint, and the posterior segment endpoint; and

a correction processing unit, configured to perform refraction correction and summation on the multiple segment lengths to obtain the eye axial length of the eye to be measured.

In one embodiment, the above apparatus for measuring eye axial length 1 further includes:

a third obtaining module 40, configured to obtain an anterior segment mark of the anterior segment endpoint and a posterior segment mark of the posterior segment endpoint according to the optical image; and

a length display module 50, configured to display an eye axial length according to the anterior segment mark and the posterior segment mark.

In one embodiment, the length display module 50 above further includes:

a mark display unit, configured to display the anterior segment mark and the posterior segment mark;

an information obtaining unit, configured to obtain position adjustment information after a user adjusts the anterior segment mark and the posterior segment mark; and

a length display unit, configured to determine the eye axial length according to the position adjustment information and display the eye axial length.

Various modules of the above apparatus for measuring eye axial length can be implemented entirely or partially by software, hardware, or a combination thereof. The above modules can be embedded in or independent of the processor in the computer device in a hardware form, or can be stored in the memory of the computer device in a software form, so that the processor can invoke and execute the operation corresponding to each of the above modules.

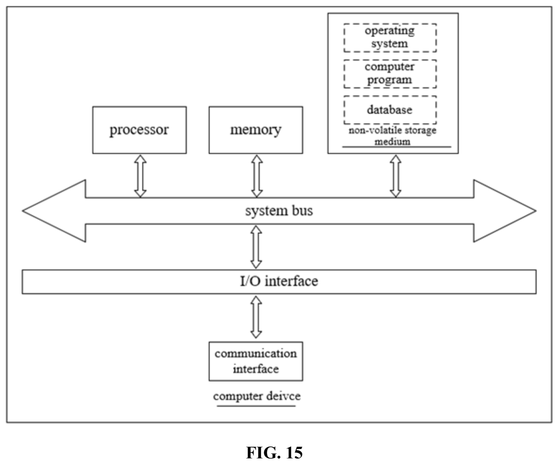

In one embodiment, a computer device is provided, the computer device can be a server, and its internal structure can be as shown in FIG. 15. The computer device includes a processor, memory, and network interface connected via a system bus. The processor of the computer device is configured to provide computing and control capabilities. The memory of the computer device includes non-volatile storage media and inner memory. The non-volatile storage media store an operating system, computer programs, and a database. The inner memory provides an environment for running the operating system and computer programs in the non-volatile storage media. The database of the computer device is configured to store eye axial length measurement data. The network interface of the computer device is configured for communication with external terminals via a network. The computer program, when executed by the processor, implements the method for measuring eye axial length.

In one embodiment, a computer device is provided, including a memory and a processor, wherein the memory stores a computer program. The processor, when executing the computer program, implements the following steps:

obtaining an optical image with an OCT optical focus aligning with a posterior segment of an eye to be measured, wherein the optical image comprises a cornea and a fovea of a retina of the eye to be measured;

obtaining an anterior segment endpoint and a posterior segment endpoint of the eye to be measured according to the optical image; and

determining an eye axial length of the eye to be measured according to the anterior segment endpoint and the posterior segment endpoint.

The provided computer device, in implementing the principles and processes of each embodiment, can refer to the descriptions in the foregoing embodiments of the method for measuring eye axial length, which are not repeated herein.

In one embodiment, a computer program product is provided, including a computer program, wherein the computer program, when executed by a processor, implements the following steps:

obtaining an optical image with an OCT optical focus aligning with a posterior segment of an eye to be measured, wherein the optical image comprises a cornea and a fovea of a retina of the eye to be measured;

obtaining an anterior segment endpoint and a posterior segment endpoint of the eye to be measured according to the optical image; and

determining an eye axial length of the eye to be measured according to the anterior segment endpoint and the posterior segment endpoint.

**INDUSTRIAL PRACTICALITY**

By applying the technical solutions of the present invention, accurate measurement of eye axial length can still be achieved for more severe ophthalmic diseases.

**Claims**

1. A method for measuring eye axial length, wherein the method comprises:

obtaining an optical image with an OCT optical focus aligning with a posterior segment of an eye to be measured, wherein the optical image comprises a cornea and a fovea of a retina of the eye to be measured;

obtaining an anterior segment endpoint and a posterior segment endpoint of the eye to be measured according to the optical image; and

determining an eye axial length of the eye to be measured according to the anterior segment endpoint and the posterior segment endpoint.

2. The method according to claim 1, wherein the step of obtaining an optical image with an OCT optical focus aligning with a posterior segment of an eye to be measured comprises:
obtaining an optical image with the OCT optical focus aligning with the retina of the eye to be measured.

3. The method according to claim 1, wherein the step of obtaining an anterior segment endpoint and a posterior segment endpoint of the eye to be measured according to the optical image comprises:
inputting the optical image into a pre-trained first neural network model to obtain the anterior segment endpoint and the posterior segment endpoint output by the first neural network model, wherein the first neural network model is trained based on first optical image samples and corresponding labels, and the labels of the first optical image samples comprise an anterior segment endpoint mark and a posterior segment endpoint mark.

4. The method according to claim 1, wherein the step of obtaining an anterior segment endpoint and a posterior segment endpoint of the eye to be measured according to the optical image comprises:

obtaining the cornea and the fovea of the retina of the eye to be measured according to the optical image;
determine the anterior segment endpoint according to the cornea; and
determining the posterior segment endpoint according to the fovea of the retina.

5. The method according to claim 4, wherein the step of obtaining the cornea and the fovea of the retina of the eye to be measured according to the optical image comprises:
inputting the optical image into a pre-trained second neural network model to obtain the cornea and the fovea of the retina output by the second neural network model, wherein the second neural network model is trained based on second optical image samples and corresponding labels, and the labels of the second optical image samples comprise a cornea mark and a fovea mark of the retina.

6. The method according to claim 4 or 5, wherein the step of determining the anterior segment endpoint according to the cornea comprises:

taking a cornea vertex of the cornea as the anterior segment endpoint; or
taking an intersection point of an OCT scanning center line and an anterior surface of the cornea as the anterior segment endpoint in response to the optical image being obtained by arranging the OCT scanning center to align with the cornea vertex and the fovea of the retina of the eye to be measured.

7. The method according to claim 4 or 5, wherein the step of determining the posterior segment endpoint according to the fovea of the retina comprises:

taking the fovea of the retina as the posterior segment endpoint; or
performing layering on the retina, obtaining a layering result of at least one retinal layer at the fovea of the retina, and determining the posterior segment endpoint according to the layering result.

8. The method according to claim 7, wherein the step of determining the posterior segment endpoint according to the layering result comprises:

selecting a target layer from the layering result and taking a target point in the target layer as the posterior segment endpoint, wherein the target layer comprises one of a retinal pigment epithelium-choroid complex layer, a Bowman's layer, and a choroid layer, and the target point is a point on a straight line where the fovea of the retina and the cornea vertex are located; or
taking an intersection point between the OCT scanning center line and the target layer in the layering result as the posterior segment endpoint in response to the optical image being obtained by arranging the OCT scanning center to align with the cornea vertex and the fovea of the retina of the eye to be measured.

9. The method according to any one of claims 1 to 8, wherein the step of determining an eye axial length of the eye to be measured according to the anterior segment endpoint and the posterior segment endpoint comprises:

obtaining an endpoint distance between the anterior segment endpoint and the posterior segment endpoint, and a correction value corresponding to a retinal thickness of the eye to be measured;
determining an initial eye axial length of the eye to be measured according to the endpoint distance and the correction value corresponding to the retinal thickness; and
performing refraction correction on the initial eye axial length to obtain the eye axial length of the eye to be measured.

10. The method according to claim 9, wherein the correction value corresponding to the retinal thickness is a preset value, a retinal thickness at the fovea of the retina, or a thickness between a retinal anterior surface at the fovea of the retina and the posterior segment endpoint.

11. The method according to claim 10, wherein the preset value is between 100 micrometers and 300 micrometers.

12. The method according to any one of claims 9 to 11, wherein the step of determining an initial eye axial length of the eye to be measured according to the endpoint distance and the correction value corresponding to the retinal thickness comprises:
taking a difference between the endpoint distance and the correction value corresponding to the retinal thickness as the initial eye axial length of the eye to be measured.

13. The method according to any one of claims 9 to 12, wherein the step of performing refraction correction on the initial eye axial length to obtain the eye axial length of the eye to be measured comprises: performing refraction correction on the initial eye axial length to obtain the eye axial length of the eye to be measured according to a preset refractive index correction coefficient.

14. The method according to claim 13, wherein the refractive index correction coefficient comprises an average refractive index correction coefficient; and the step of performing refraction correction on the initial eye axial length to obtain the eye axial length of the eye to be measured according to a preset refractive index correction coefficient comprises:

calculating an eye axial length $L_2$ of the eye to be measured through a formula:

$$L_2 = k \times L_1,$$

where k represents the average refractive index correction coefficient and $L_1$ represents the initial eye axial length.

15. The method according to claim 14, wherein the average refractive index correction coefficient is between 1.33 and 1.45.

16. The method according to any one of claims 1 to 8, wherein the step of determining an eye axial length of the eye to be measured according to the anterior segment endpoint and the posterior segment endpoint comprises:

obtaining multiple structural layers of the eye to be measured between the anterior segment endpoint and the posterior segment endpoint according to the optical image, wherein the multiple structural layers comprise an anterior surface and a posterior surface of the cornea, an anterior surface of a lens, and a posterior surface of the lens; obtaining multiple segment lengths corresponding one-to-one to the cornea, an anterior chamber, the lens, and a vitreous body of the eye to be measured in an eye axial direction according to the multiple structural layers, the anterior segment endpoint, and the posterior segment endpoint; and performing refraction correction and summation on the multiple segment lengths to obtain the eye axial length of the eye to be measured.

17. The method according to any one of claims 1 to 16, wherein the method further comprises:

obtaining an anterior segment mark of the anterior segment endpoint and a posterior segment mark of the posterior segment endpoint according to the optical image; and displaying the eye axial length according to the anterior segment mark and the posterior segment mark.

18. The method according to claim 17, wherein the step of displaying the eye axial length according to the anterior segment mark and the posterior segment mark comprises:

displaying the anterior segment mark and the posterior segment mark; obtaining position adjustment information after a user adjusts the anterior segment mark and the posterior segment mark; and determining the eye axial length according to the position adjustment information and displaying the eye axial length.

19. An apparatus for measuring eye axial length, wherein the apparatus comprises:

a first obtaining module, configured to obtain an optical image with an OCT optical focus aligning with a posterior segment of an eye to be measured, wherein the optical image comprises a cornea and a fovea of a retina of the eye to be measured; a second obtaining module, configured to obtain an anterior segment endpoint and a posterior segment endpoint of the eye to be measured according to the optical image; and a length determining module, configured to determine an eye axial length of the eye to be measured according to the anterior segment endpoint and the posterior segment endpoint.

20. A computer device, comprising a memory and a processor, wherein the memory stores a computer program, and the processor, when executing the computer program, implements the method for measuring eye axial length according to any one of claims 1 to 18.

**FIG. 1**

| | |
|---|---|
| obtaining an optical image with an OCT optical focus aligning with a posterior segment of an eye to be measured | S201 |
| obtaining an anterior segment endpoint and a posterior segment endpoint of the eye to be measured according to the optical image | S202 |
| determining an eye axial length of the eye to be measured according to the anterior segment endpoint and the posterior segment endpoint | S203 |

**FIG. 2**

**FIG. 3**

**FIG. 4**

**FIG. 5**

**FIG. 6**

| obtaining a corneal apex and a fovea of the retina of the eye to be measured according to the optical image | S301 |
|---|---|
| taking the corneal vertex as the anterior segment endpoint | S302 |
| taking the fovea of the retina as the posterior segment endpoint | S303 |

**FIG. 7**

obtaining an endpoint distance between the anterior segment endpoint and the posterior segment endpoint, and a correction value corresponding to a retinal thickness of the eye to be measured — S401

determining an initial eye axial length of the eye to be measured according to the endpoint distance and the correction value corresponding to the retinal thickness — S402

performing refraction correction on the initial eye axial length to obtain the eye axial length of the eye to be measured — S403

**FIG. 8**

**FIG. 9**

**FIG. 10**

| | |
|---|---|
| obtaining an optical image with an OCT optical focus aligning with a retina of the eye to be measured | S501 |
| obtaining a corneal apex and a fovea of the retina of the eye to be measured according to the optical image | S502 |
| taking the corneal vertex as the anterior segment endpoint | S503 |
| taking the fovea of the retina as the posterior segment endpoint | S504 |
| obtaining an endpoint distance between the anterior segment endpoint and the posterior segment endpoint, and a retinal thickness of the eye to be measured | S505 |
| taking a difference between the endpoint distance and the retinal thickness as the initial eye axial length of the eye to be measured | S506 |
| performing refraction correction on the initial eye axial length to obtain the eye axial length of the eye to be measured according to the refractive index correction coefficient | S507 |
| obtaining an anterior segment mark of the anterior segment endpoint and a posterior segment mark of the posterior segment endpoint according to the optical image | S508 |
| displaying the anterior segment mark and the posterior segment mark | S509 |
| obtaining position adjustment information after a user adjusts the anterior segment mark and the posterior segment mark | S510 |
| determining the eye axial length according to the position adjustment information and displaying the eye axial length | S511 |

**FIG. 11**

apparatus for measuring eye axial length — 1

first obtaining module — 10

second obtaining module — 20

length determining module — 30

**FIG. 12**

apparatus for measuring eye axial length — 1

second obtaining module — 20

first obtaining unit — 21 ——— first obtaining module — 10

first determining unit — 22

second determining unit — 23 ——— length determining module — 30

**FIG. 13**

apparatus for measuring eye axial length — 1

second obtaining module — 20

first obtaining unit — 21 ——— first obtaining module — 10

first determining unit — 22

second determining unit — 23

length determining module — 30

second obtaining unit — 31

third determining unit — 32

fourth determining unit — 33

**FIG. 14**

**FIG. 15**

## INTERNATIONAL SEARCH REPORT

| International application No. |
|---|
| **PCT/CN2024/098173** |

| **A.** | **CLASSIFICATION OF SUBJECT MATTER** |
|---|---|

A61B3/10(2006.01)i; A61B3/14(2006.01)i; A61B3/00(2006.01)i

According to International Patent Classification (IPC) or to both national classification and IPC

| **B.** | **FIELDS SEARCHED** |
|---|---|

Minimum documentation searched (classification system followed by classification symbols)

IPC: A61B

Documentation searched other than minimum documentation to the extent that such documents are included in the fields searched

Electronic data base consulted during the international search (name of data base and, where practicable, search terms used)

CNTXT, ENTXTC, ENTXT, DWPI, CNKI: 视微影像（河南）科技有限公司, 臧璇, 王嘉囡, 陈军, 吴之林, 眼轴, 视轴, 前后轴, AL, 眼前段, 眼前节, 眼球前节, 前眼部, 前眼段, 视网膜, 视網膜, 網膜, 眼后段, 眼底, 眼后节, 眼球后节, 眼底, 后眼部, 眼内, 眼前后节, 光学相干断层扫描, 光学相干层析成像, OCT, 角膜, 焦点, 聚焦, 中央凹, 中心凹, 厚度, 长度, 神经网络, 深度学习, 标签, ocular axis, visual axis, axial length, AL, anterior segment, anterior ocular, retina, posterior segment, eyeground, posterior ocular, intraocular, anterior posterior segment, optical coherence tomography, OCT, cornea, focus, fovea, thickness, length, neural network, deep learning, label

| **C.** | **DOCUMENTS CONSIDERED TO BE RELEVANT** |
|---|---|

| Category* | Citation of document, with indication, where appropriate, of the relevant passages | Relevant to claim No. |
|---|---|---|
| PX | CN 116636806 A (SVISION IMAGING HENAN TECHNOLOGY CO., LTD.) 25 August 2023 (2023-08-25)<br>description, paragraphs [0058]-[0213], and figures 1-14 | 1-20 |
| Y | CN 114903426 A (SVISION IMAGING HENAN TECHNOLOGY CO., LTD.) 16 August 2022 (2022-08-16)<br>description, paragraphs [0063]-[0183], and figures 1-12 | 1-20 |
| Y | CN 115399728 A (JIAXING RESEARCH INSTITUTE, ZHEJIANG UNIVERSITY et al.) 29 November 2022 (2022-11-29)<br>description, paragraphs [0057]-[0099], and figures 1-6 | 1-20 |
| Y | CN 114926447 A (BEIJING BAIDU NETCOM SCIENCE AND TECHNOLOGY CO., LTD.) 19 August 2022 (2022-08-19)<br>description, paragraphs [0025]-[0097], and figures 1-8 | 3, 5-18, 20 |

| ✓ | Further documents are listed in the continuation of Box C. | | ✓ | See patent family annex. |
|---|---|---|---|---|

| * | Special categories of cited documents: |
|---|---|
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **22 August 2024** | **28 August 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**

| International application No. |
|---|
| **PCT/CN2024/098173** |

## C. DOCUMENTS CONSIDERED TO BE RELEVANT

| Category* | Citation of document, with indication, where appropriate, of the relevant passages | Relevant to claim No. |
|---|---|---|
| Y | CN 114947728 A (NORTHEASTERN UNIVERSITY AT QINHUANGDAO) 30 August 2022 (2022-08-30)<br>description, paragraphs [0003] and [0027]-[0051], and figures 1-3 | 9-18, 20 |
| A | CN 103976708 A (SHENZHEN CERTAINN TECHNOLOGY CO., LTD. (MOPTIM)) 13 August 2014 (2014-08-13)<br>entire document | 1-20 |
| A | CN 110522406 A (GUANGZHOU HAOKANG BIOTECHNOLOGY CO., LTD.) 03 December 2019 (2019-12-03)<br>entire document | 1-20 |
| A | CN 113558563 A (ZHEJIANG UNIVERSITY et al.) 29 October 2021 (2021-10-29)<br>entire document | 1-20 |
| A | CN 116194032 A (ALCON INC.) 30 May 2023 (2023-05-30)<br>entire document | 1-20 |
| A | JP 2020081469 A (TOPCON CORP.) 04 June 2020 (2020-06-04)<br>entire document | 1-20 |
| A | US 2013242259 A1 (CARL ZEISS MEDITEC AG.) 19 September 2013 (2013-09-19)<br>entire document | 1-20 |

Form PCT/ISA/210 (second sheet) (July 2022)

**INTERNATIONAL SEARCH REPORT**
Information on patent family members

International application No.

**PCT/CN2024/098173**

| Patent document cited in search report | | | Publication date (day/month/year) | Patent family member(s) | | | Publication date (day/month/year) |
|---|---|---|---|---|---|---|---|
| CN | 116636806 | A | 25 August 2023 | None | | | |
| CN | 114903426 | A | 16 August 2022 | CN | 114903426 | B | 08 September 2023 |
| CN | 115399728 | A | 29 November 2022 | None | | | |
| CN | 114926447 | A | 19 August 2022 | CN | 114926447 | B | 29 August 2023 |
| CN | 114947728 | A | 30 August 2022 | None | | | |
| CN | 103976708 | A | 13 August 2014 | None | | | |
| CN | 110522406 | A | 03 December 2019 | None | | | |
| CN | 113558563 | A | 29 October 2021 | None | | | |
| CN | 116194032 | A | 30 May 2023 | AU | 2021350571 | A1 | 09 March 2023 |
| | | | | EP | 4216798 | A1 | 02 August 2023 |
| | | | | WO | 2022064337 | A1 | 31 March 2022 |
| | | | | US | 2022095909 | A1 | 31 March 2022 |
| | | | | CA | 3191958 | A1 | 31 March 2022 |
| | | | | JP | 2023542671 | A | 11 October 2023 |
| JP | 2020081469 | A | 04 June 2020 | JP | 7186587 | B2 | 09 December 2022 |
| US | 2013242259 | A1 | 19 September 2013 | DE | 102010046500 | A1 | 29 March 2012 |
| | | | | WO | 2012038011 | A1 | 29 March 2012 |
| | | | | EP | 2618720 | A1 | 31 July 2013 |
| | | | | US | 9364144 | B2 | 14 June 2016 |

Form PCT/ISA/210 (patent family annex) (July 2022)

**EP 4 710 840 A1**

REFERENCES CITED IN THE DESCRIPTION

**Patent documents cited in the description**

- CN 202310673845